# EUROPEAN PATENT APPLICATION

(11) **EP 1 582 512 A1**
(43) Date of publication of application: **05.10.2005**
(21) Application number: 04007767.9
(22) Date of filing: 31.03.2004
(51) Int. Cl.: C07C 37/00, C07C 39/11

(54) **Process for obtaining hydroxytyrosol from olive leaves extracts**

(71) Applicant: Cognis IP Management GmbH, 40589 Düsseldorf (DE)
(72) Inventor: Beverungen, Carsten, 40591 Düsseldorf (DE); Rull Prous, Santiago, 08034 Barcelona (ES); Kempers, Peter Dr., 41516 Grevenbroich (DE); Buchwald-Werner, Sybille Dr., 40589 Düsseldorf (DE)

(57) **Abstract**

The present invention refers to a new process for obtaining hydroxytyrosol, by
(a) subjecting extracts of olive leaves to hydrolysis in order to obtain an aqueous mixture which is rich in hydroxytyrosol,
(b) mixing the intermediate thus obtained with a micro porous resin in order to adsorb the hydroxytyrosol on said matrix and to keep the unwanted by-products within the aqueous phase, and finally
(c) separating the matrix from the aqueous phase and desorbing the hydroxytyrosol by means of a suitable solvent.

## Description

### Object of the invention

The present invention belongs to the area of antioxidants and refers to a new process for obtaining hydroxytyrosol from the extracts of olive leaves.

### State of the art

The olive tree is a culturally significant plant for the Mediterranean region. Olive leaves *(Olea europaea L.)* have been used for thousands of years as a natural antioxidant. Oleuropein is a phenolic substance occurring in olive fruits and olive leaves, that is well known for its antimicrobial, anti-inflammatory and antioxidant effects. Hydroxytyrosol ((3,4-dihydroxyphenyl)-ethanol) is a cleavage product of oleuropein and shows also antioxidative properties. In "in vitro" tests, the antioxidant efficiency of hydroxytyrosol was monitored higher than oleuropein, tocopherol and ascorbic acid. Hydroxytyrosol can be synthesized by chemical hydrolysis of oleuropein. In this reaction, elenolic acid glycoside (Oleoside-11-methylester) is build as by-product. The positive properties of olive derived phenols are broadly mentioned in the literature. Micol et al. describe the antiviral character of olive leaf extract, containing 20 % oleuropein. [V. Micol, et. al., **New applications of herbal extracts for functional food and pharmaceuticals. Part 2.AgroFOOD Industry HI-Tech, November/December 2003, p14-16].** The antioxidative properties of phenolic ingredients in olives, especially in oleuropein and it's cleavage products, were described by H. Schmandke. **[Ernaehrungs-Umschau 48 (2001) Heft 12, p490-492].**

Patent literature contains numerous references that deal with the extraction of oleuropein and hydroxytyrosol from olive leaves. For example **WO 02/18310 A1, US 2002/0198415 A1, WO 2004/005228 A1, US 6,416,808** and **US 2002/0058078 A1** claim an acidic hydrolysis of olive vegetation water for 2 to 12 month until at least 90 % of the present oleuropein has been converted. A method for extraction of antioxidant compositions from olives, olive pulps, olive oil and oil mill waste water is described by Usana Inc. patents **US 6,361,803** and **WO 01/45514** A1 and by Workman Nydegger & Seeley patent application **US 2002/0004077 A1.** These documents also claim the purification of antioxidant components from waste water by a solid matrix, like polymeric, styrenic resins. These resins could be of the type of Amberlite® resins or Duolite® resins.

The chemoenzymatic hydrolysis of oleuropein was described by Briante et al.. In a first step oleuropein was converted to it's aglycon by a hyperthermophilic β-glycosidase and was then chemically hydrolysed to hydroxytyrosol and elenolic acid glycoside. [R. Briante et. al., **Journal of Biotechnology 93 (2002), p109-119;** ***ibid.*** **77 (2000) p275-286].** A method to purify hydroxytyrosol from olive waste by extraction and chromatography was described by Fernandez-Bolanos et al.. **[J. Agric. Food Chem. 2002, 50, p6804-6811**].

None of the processes known from the state of the art, however, provides Hydroxytyrosol both in high concentration and in high purity. Therefore, the object of the present invention was to develop a simple and fast process for obtaining a concentrate with a high content of hydroxytyrosol, which is substantially free (less than 0.5 % b.w.) of oleuropein and elenolic acid glycoside (oleoside-11-methylester).

### Description of the invention

The present invention claims a new process for obtaining hydroxytyrosol, by
(a) subjecting extracts of olive leaves to hydrolysis in order to obtain an aqueous mixture which is rich in hydroxytyrosol,
(b) mixing the intermediate thus obtained with a micro porous resin in order to adsorb the hydroxytyrosol on said matrix and to keep the unwanted by-products within the aqueous phase, and finally
(c) separating the matrix from the aqueous phase and desorbing the hydroxytyrosol by means of a suitable solvent.

Surprisingly, it has been found, that hydroxytyrosol of high purity can be isolated from olive leaf extracts according to the invention by means of hydrolysis and adsorption. Originating from olive leaf extracts, a hydrolysation product can be produced by acidic or basic hydrolysis, that contains a high level of hydroxytyrosol and is substantially free of Oleuropein. The purification methods described in the state of the art allow an enrichment of the product in several steps. Moreover, it has been found that hydroxytyrosol from olive leaf extract can be purified to a high content in only a singly step by treating the hydrolysation product thus obtained with micro porous resins, especially with a resin of the Lewatit® type, to ensure adsorption of the hydroxytyrosol, while by-products and impurities remain in the aqueous phase. Subsequently, the hydroxytyrosol can be liberated from the matrix by simply washing it with a suitable solvent.

### Hydrolysis

Although also products from olive waste water removal can be used as a starting material, it is desirable to start the process with extracts from olive leaves, since they show a significantly higher content of oleuropein. Such concentrates are available in the market, e.g. from Cognis Iberia S.L., comprising between 20 and 80 % b.w. oleuropein.

The hydrolysis of oleuropein can be carried out according to the methods known from the state of the art, that means either under alkaline or acidic conditions. Preferably, the reactions are carried out with 10 % (w/w) olive leaf extract in buffer or water, at pH-values between pH 1 and 11. The best hydrolysis was achieved at acidic conditions, at a pH-value of pH 1 to 3 and a temperature of 70 to 90°C. Under these conditions a quantitative hydrolysis of the present oleuropein to hydroxytyrosol and elenolic acid glycoside can be achieved within 1 to 10, preferably 4 to 5 hours reaction-time. The quantitative analysis of oleuropein and hydroxytyrosol was performed by HPLC and GC.

### Adsorption

The downstream processing of hydroxytyrosol was carried out by adsorption. Hydroxytyrosol was trapped on a solid micro porous resin matrix. This has the major advantage that other substances like oleuropein or elenolic acid glycoside are not adsorbed, thus a separation and purification is easily effected. Desorption was achieved by washing the matrix with a solvent. To concentrate the product, it is suggested to remove the solvent, e.g. by means of vacuum evaporation. The best method for the downstream-processing was the use of the adsorber Lewatit® VP OC 1163 or the food grade adsorber Lewatit® S 7768 at a pH-value of 5 to 9, preferably at 7.7 and a temperature of 30 to 60 °C, preferably about 45 °C. Desorption can be carried out with a suitable solvent, like e.g. ethanol or methanol.

### Examples

### Example 1

### HPLC-Method for oleuropein- and hydroxytyrosol-analysis

Quantitative analysis of oleuropein and hydroxytyrosol was performed by HPLC, using the µRPC-C2/C18-ST-4.6/100 column by Amersham Pharmacia Biotech, particle size 3 µm, length 100 mm, diameter 4.6 mm and the UV-Detector LTV-900 by Amersham Pharmacia Biotech. A gradient from 0 % to 20 % acetonitrile in water (containing 0,1 % acetic acid) was carried out in 6.2 min (3 column volumes). Afterwards a more flat gradient from 20 % to 45 % acetonitrile was carried out within 20.8 min (10 column volumes). The flow rate was 0.8 mL/min. Detection was performed at 234 and 285 nm.

### Example 2

### GC-Method for oleuropein- and hydroxytyrosol-analysis

Quantitative analysis of oleuropein and hydroxytyrosol was also performed by gas chromatography, using a DB-5HT column (15m × 320µm × 0.1µm) by Agilent Technologies. A 40 minutes linear gradient from 80°C to 360°C (7°C/min) was used. Detection was performed by a flame ionisation detector, FID (Agilent Technologies).

### Example 3

### Hydrolysis of olive leaf extract at pH 1.5

2.5 g purified olive extract (44.7 % oleuropein) was mixed with 50 mL of de-ionised water. 0.1 M hydrochloric acid was added, until pH 1.5 was reached (approx. 10 mL). This mixture was put into a heated oil bath at T=90°C and was mixed by a magnetic stirrer at n=200 rpm. During the reaction time of t=24 h samples were taken for HPLC-analysis. The details can be taken from table 1. After 4 hrs of reaction time, 66.7 % hydroxytyrosol was produced. No oleuropein was detected.

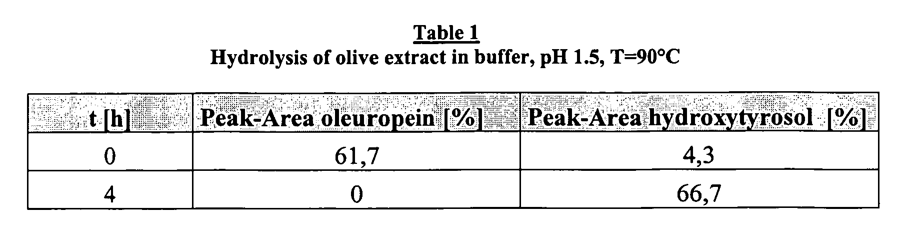

### Comparative Example C1

### Hydrolysis of olive leaf extract at pH 5.5, T=70°C

2.5 g purified olive extract (44.7 % oleuropein), mixed in 50 mL of 0.1 M acetate-buffer (pH 5.5), was prepared in a 100 mL flask. This flask was put into a heated oil bath at T=70°C and was mixed by a magnetic stirrer at n=200 rpm. During the reaction time of t=189 hrs, samples were taken for HPLC-analysis. The details can be taken from table 2. The yield after 189 hrs reaction time is 12.4 % hydroxytyrosol.

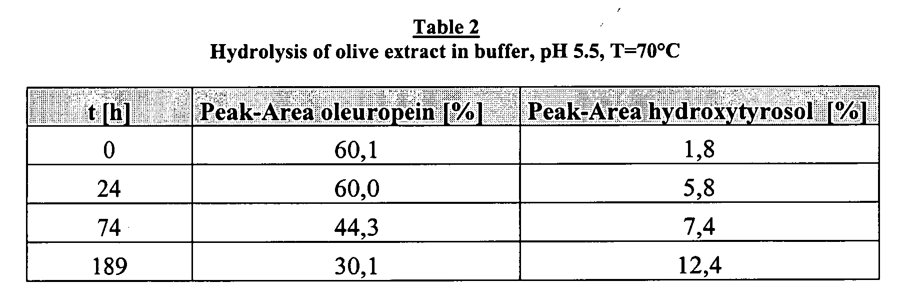

### Comparative Example C2

### Hydrolysis of olive leaf extract at pH 3.6, T=60°C

2.5 g purified olive extract (44.7 % oleuropein), mixed in 50 mL of de-ionised water, was prepared in a 100 mL flask. The pH-value of this mixture was pH 3.6. The flask was put into a shaker at T=60°C and was mixed at n=200 rpm. During the reaction time of t=189 h, samples were taken for HPLC-analysis. The details can be taken from table 3. The yield after 189 hrs reaction time is 57.1 % hydroxytyrosol.

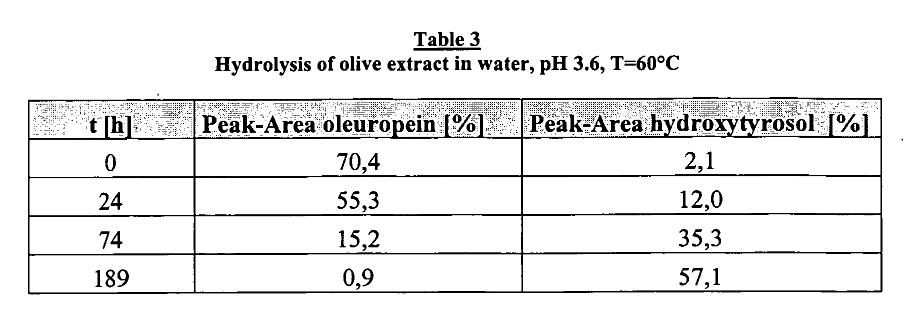

### Comparative Example C3

### Hydrolysis of olive leaf extract at pH 9.0, T=60°C

10 g purified olive extract (44.7 % oleuropein), mixed in 100 mL of 1 M borate-buffer (pH 9), was prepared in a 250 mL flask. This flask was put into a thermo shaker at T=60°C and was shaked at n=200 rpm. During the reaction time of t=189 h, samples were taken for HPLC-analysis. In table 4 the course of the reaction is shown. The yield after 189 hrs reaction time is 44.5 % hydroxytyrosol.

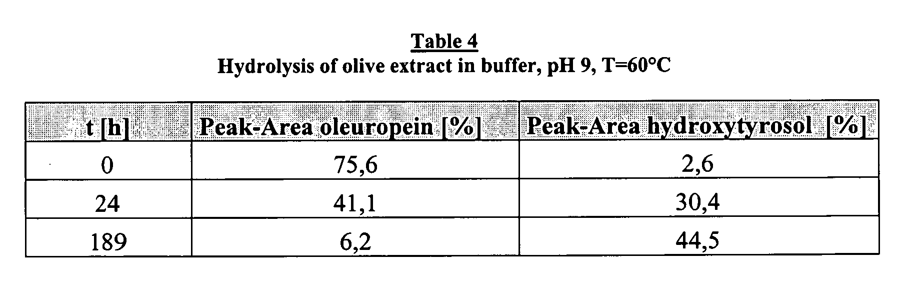

### Example 4

### Downstream processing of hydroxytyrosol

The adsorption- and desorption method was tested with a product from acidic hydrolysis. After hydrolysis, the reaction media was adjusted with sodium hydroxide to pH 7.7. Afterwards 10 w/w % Lewatit VP OC 1163 was added. This mixture was allowed to shake for 1 hour at room temperature. After this time, the adsorber was filtrated by a nutsch filter. Afterwards the tenfold amount of ethanol (v/w) was added to the adsorber and the mixture was shaken for 30 minutes at T=30°C. Subsequently the adsorber was filtrated by a nutsch filter and the solvent was evaporated under vacuum. The purified product was analysed by HPLC and GC. The purity of the hydroxytyrosol was 93 %.

### Comparative Example C4

### Downstream processing of hydroxytyrosol

The adsorption- and desorption method was tested with a product from acidic hydrolysis. After hydrolysis, the reaction media had a pH-value of 1.3. Afterwards 10 w/w % Amberlite® XAD 16 HP, Amberlite® XAD 1180, Amberlite® XAD 7 HP or Lewatit® VP OC 1064 MDPH were added. This mixture was allowed to shake for 1 hour at room temperature. After this time, the adsorber was filtrated by a nutsch filter. Afterwards the tenfold amount of methanol (v/w) was added to the adsorber and the mixture was shaken for 30 minutes at T=30°C. Subsequently the adsorber was filtrated by a nutsch filter and the filtrate was analysed by HPLC and GC. No hydroxytyrosol could be detected.

## Claims

1. Process for obtaining hydroxytyrosol, **characterised in that**
(a) extracts of olive leaves are subjected to hydrolysis in order to obtain an aqueous mixture which is rich in hydroxytyrosol,
(b) the intermediate thus obtained is mixed with a micro porous resin in order to adsorb the hydroxytyrosol on said matrix and to keep the unwanted by-products within the aqueous phase, and finally
(c) the matrix is separated from the aqueous phase and the hydroxytyrosol is desorbed by means of a suitable solvent.

2. Process according to claim 1, **characterised in that** said extracts of olive leaves show a content of oleuropein between 20 and 80 % b.w.

3. Process according to claims 1 and/or 2, **characterised in that** said hydrolysis is carried out at a pH-value of 1 to 3 and 7 to 11.

4. Process according to any of the claims 1 to 3, **characterised in that** said hydrolysis is carried out at a temperature of from 70 to 100 °C.

5. Process according to any of the claims 1 to 4, **characterised in that** said hydrolysis is carried out over a period of 1 to 10 hours.

6. Process according to any of the claims 1 to 5, **characterised in that** said one uses Lewatit® VP OC 1163 or Lewatit® S 7768 as the micro porous resin matrix.

7. Process according to any of the claims 1 to 6, **characterised in that** the adsorption is carried out at a pH-value of 5 to 9.

8. Process according to any of the claims 1 to 7, **characterised in that** the adsorption is carried out a temperature of from 20 to 60 °C.

9. Process according to any of the claims 1 to 8, **characterised in that** said the adsorption is carried out using either in water or an aqueous buffer phase.

10. Process according to any of the claims 1 to 9, **characterised in that** the hydroxytyrosol is liberated from the matrix by using a lower alcohol as a solvent.
